# EUROPÄISCHE PATENTSCHRIFT

(11) **EP 3 292 846 B1**
(45) Veröffentlichungstag und Bekanntmachung des Hinweises auf die Patenterteilung: **25.02.2026**
(21) Anmeldenummer: 17193100.9
(22) Anmeldetag: 31.10.2006
(51) Int. Cl.: A61F 13/01, A61F 13/00, A61F 13/06

(54) **ABSORPTIONSKÖRPER ZUR ANBRINGUNG AN MENSCHLICHE ODER TIERISCHE HAUTOBERFLÄCHEN**
ABSORPTION BODY FOR ATTACHMENT TO HUMAN OR ANIMAL SKIN SURFACES
CORPS D'ABSORPTION DESTINÉ À ÊTRE APPLIQUÉ SUR LA SURFACE CUTANÉE D'UN ÊTRE HUMAIN OU D'UN ANIMAL

(30) Priorität: 02.11.2005 DE 202005017211 U
(43) Veröffentlichungstag der Anmeldung: 14.03.2018
(62) Teilanmeldung aus: 11160118.3
(73) Patentinhaber: BSN medical GmbH, 22761 Hamburg (DE)
(72) Erfinder: Riesinger, Birgit, 48149 Münster (DE)
(74) Vertreter: Cohausz & Florack

(56) Entgegenhaltungen:
- EP-A1- 0 413 251
- DE-A1- 10 059 439
- DE-U1- 202004 017 052
- US-A- 5 998 032

## Beschreibung

Die Erfindung betrifft eine Mischung aus einer Menge stark osmotisch wirksamer Substanzen, die auf der Basis von Acrylsäure hergestellt sind, mit einer Menge osmotisch vergleichsweise schwachen oder osmotisch inaktiven Substanzen zur Anwendung in einem Verfahren für die Behandlung chronischer Wunden, der Ödemtherapie oder der Behandlung des Ulcus cruris, zum Aufsaugen von schädlichen Proteasen, wobei die Mischung in einer inneren Lage vorliegt, die im Wesentlichen aus der Mischung besteht, wobei die innere Lage von einer äußeren Umhüllung, die durchlässig für flüssige Stoffe ist, umschlossen ist, wobei die innere Lage und die äußere Umhüllung einen Absorptionskörper zur Anbringung an menschliche oder tierische Hautoberflächen im Bereich von Wunden bilden.

Ein Absorptionskörper ist der DE 100 59 439 der Anmelderin zu entnehmen. Der bekannte Absorptionskörper hat sich in der Praxis gut bewährt, jedoch besteht der Bedarf nach Verstärkung der Saugleistung, insbesondere bei mittel bis stark sezernierenden, infizierten Wunden, aus denen das Wundexsudat aus der Wundgrundtiefe effektiver herausgezogen werden kann. Ein weiterer Absorptionskörper ist aus US 5998032 bekannt.

Diese Aufgabe ist durch die Mischung gemäß Anspruch 1 gelöst. Weitere Ausführungsformen sind Gegenstand der abhängigen Ansprüche.

Bei dem Absorptionskörper enthaltend die erfindungsgemäße Mischung ist die innere Lage derart mit osmotisch wirksamen Substanzen aufgefüllt, dass auf eine Wunde mit den darin enthaltenen Wundflüssigkeiten ein osmotischer Druck ausübbar ist, über den die Wundflüssigkeit dem zu behandelnden Organismus entziehbar ist und somit sowohl in der oberflächigen Wundregion als auch in der Gewebetiefe eine interstitielle Normohydratation von Gewebe unterstützbar ist, indem körpereigene Flüssigkeiten in ihrer Flussrichtung zur Hautoberfläche des Patienten in den Absorptionskörper gelenkt sind.

Erfindungsgemäß ist die innere Lage so beschaffen, dass die Flächenmasse wenigstens 420g/m² ist, wobei die Flächenmasse des darin gleichmäßig verteilten Anteils der osmotisch wirksamen Substanzen wenigstens 200g/m² beträgt.

Die Begriffe der Behandlung chronischer Wunden und der Ödemtherapie sind oftmals nicht zu trennen, da entzündliche Prozesse, infektiöse Ereignisse und Leckagen von Gefäßen bei der einen wie der anderen Art auftreten.

Tritt ödematöse, auf dem Boden pathologischer Prozesse entstehende Flüssigkeit in die Zellen eines Gewebes ein, so werden diese Zellen komprimiert. Ihr Abstand zu ernährenden oder abtransportierenden Gefäßen wächst, Diffusionsprozesse erschweren sich, Stoffwechselprodukte reichem sich an und Sauerstoff wird rar. Hinzu kommt, dass Stoffwechselprodukte aus sterbenden Zellen austreten, so dass Zuckerabbaustoffe, wie Milchsäure (Laktat aus der Glycolyse) oder auch Zitronensäure, sich ansammeln und einen aktiven Abbau von Kollagen herbeiführen und Gewebe zerstören.

Hypothesen zufolge unterliegt dieser Abbau von Gewebe, der einer Wundentstehung gleichkommt, einer Vielzahl weiterer Prozesse. Aktivierte Leukozyten, durch Fibrin verklebte Wachstumsfaktoren (Growth factor trap hypothesis) und hypoxische Areale unterstützen den Gewebeabbau.

Die Ursachen der störenden Potenz liegen im Vorhandensein von Überwässerung und langen Stehzeiten von Wasser in Gewebe. Ein Therapieansatz, der sich dieser Erkenntnis zugewandt hat, ist die Vakuumtherapie, bei der mittels abgeschlossener Systeme die Wundregion spezifischen Unterdrücken ausgesetzt wird. Die Heilungserfolge sind beeindruckend.

Nachteilig ist, dass dieser Therapieansatz ein sehr teurer, aufwändiger und Apparate erfordernder Vorgang ist. Es ist daher vorteilhaft, analoge Mechanismen über andere physikalische Gesetzmäßigkeiten der Wundregion zu applizieren, beispielsweise über einen Verband umfassend die erfindungsgemäße Mischung mit hohem osmotischem Unterdruck. Überdimensionierte Mengen an wasserspeichernden Polymeren, z. B. 200g/m², erfüllen diese Aufgabe und machen sich zunutze, dass Wassermoleküle nur unter Aufbringung hoher Trennkraft den Kontakt zu anderen Wassermolekülen aufgeben. Der Umstand, dass ein so kleines Molekül wie Wasser bis auf 100°C erhitzt werden muss, um diese Trennung zu erreichen, bezeugt dieses.

Über den Sog an oberflächlich gelegenen Wassermolekülen werden demnach auch Wassermoleküle in tiefen Gewebeschichten erreicht, und dort die überschüssigen wässrigen Exsudate abgesaugt. Kohäsionskräfte des Wassers erlauben die kettenartige Entziehung von Wassermolekülen bis hin zum Entstehungsort der Ödeme.

Mit diesem Sog wird eine Vielzahl von Vorzügen erreicht. Kollagen abbauende Stoffe, wie Zitronen- oder Milchsäure, die im Ödem vorhanden sind und dort die Wunde aktiv erhalten, werden entzogen. Diffusionsprozesse werden wieder leichter, die Zellen erhalten Sauerstoff, Bausteine und Mediatoren wie Wachstumsfaktoren. Die Kollagensynthese kann beginnen.

Unter Ausnutzung des unerwünschten Exsudats als Trägersubstanz und als Spülmittel der Wundregion, werden im Exsudat befindliche Stoffe durch mehrere Gewebeschichten hindurch gespült und reinigen die Schichten der Wunde.

Bekannte Wundauflagen mit superabsorbierenden Substanzen haben die Eigenschaft, austretendes Wasser zu binden, um zu vermeiden, dass es auffällig austritt. Zwar wird eine "Tiefenwirkung" erwähnt, aber nicht definiert. Dagegen wird durch den vorliegenden Absorptionskörper umfassend die erfindungsgemäße Mischung z. B. beim Ulcus cruris (VLE) eine Tiefenwirkung bis hin zur insuffizienten Vene und damit zum perivenösen Gewebe und dessen Ödem erreicht. Über einen Quellprozess im Absorptionskörper wird eine Adaptation an den Wundboden im Sinne einer Wundmorphologieadapatation erreicht, da die vollgesogenen superabsorbierenden Granulate verschieblich sind.

Der Absorptionskörper umfassend die erfindungsgemäße Mischung kann in einem Verband vorliegen als Kombinationsprodukt. Mit zusätzlichen Taschen oder Umhüllungen mit perforierten Folien im Sinne von Wunddistanzgittern kann der Absorptionskörper eingelegt sein. Hier kann er auch benachbart mit anderen Verbandsstoffen, wie Schaumverbänden, Alginaten, hydrophilen Fasern, Polyhexaniden und Trägerstoffen, CMC (Carboxymethylzellulose), hydrophilen Fasern, Hydrokolloiden, Lipokolloiden, Honig, Aktivkohle, Silber, Cellulose, Pharmaka, Hydrogelen, Detergentien wie Tensiden und Poloxameren und deren Trägerstoffe, anderen superabsorberhaltigen Körpern oder Vermischungen derartiger Vorrichtungen verwendet werden. Abhängig von der Einsatzweise kann das Produkt mit einer Seite zur Wunde gelegt werden, bei der der Absorptionskörper mittelbaren Kontakt zur Wunde hat; auch kann er mit der anderen Verbandstoffart zur Wunde gerichtet sein, wobei hier die Verbandstoffart einen Durchflusskörper bildet, durch den der Absorptionskörper die Exsudate hindurch zieht und so dessen Absorptionskapazität vergrößert und dessen Liegedauer auf der Wunde verlängert.

Die Umhüllung kann aus mehr als einer Folie oder Abdeckung bestehen, beispielsweise dergestalt, dass einerseits ein Nonwoven, andererseits ein wasserfestes oder wasserdampfdurchlässiges Backsheet vorliegt. Eine andere Möglichkeit ist einerseits ein dreidimensional ausgeformtes Wunddistanzgitter und andererseits eine Abdeckung mit besonderen Funktionen, wie das Tragen von Aktivkohle oder Antiinfektiva etc., zu verwenden. Zwei homogene Abdeckungen oder die Vermischung unterschiedlicher Abdeckungen sind möglich.

Die Umhüllung, bestehend aus einem oder mehreren Zuschnitten soll bevorzugt verklebt, Ultraschall verschweißt, thermisch generiert oder mechanisch vollzogen sein. Bevorzugt bildet die Naht nicht die Außenkante des Produktes, sondern belässt einen nicht vernähten Materialanteil, der ein- oder mehrblättrig flexible und weiche Produktkanten hat. Diese Umhüllung kann im Umfang teils oder ganz verschlossen sein, an wenigstens einer Länge offen bleiben oder Umschlagsfalten aufweisen.

Neben einer räumlichen Nachbarschaft von Produkten neben dem Absorptionskörper innerhalb einer Umhüllung können die genannten Stoffe, wie Schaumverbände, Alginate, hydrophile Fasern, Polyhexanide und Trägerstoffe, CMC, Hydrokolloide, Lipokolloide, Honig, Aktivkohle, Silber, Cellulose, Pharmaka, Hydrogele, Detergentien wie Tenside und Poloxamere und deren Trägerstoffe, andere superabsorberhaltigen Körper oder Vermischungen derartiger Stoffe im Absorptionskörper selbst vorliegen, beispielsweise dergestalt, dass die Superabsorbergranulate in Stoffe dieser Art eingearbeitet sind, auch zusätzlich zu anderen Lagen, die die Superabsorber-Teilchen oder andere osmotisch aktive Stoffe enthalten.

Offenbart wird die Einarbeitung von superabsorbierenden Substanzen in hydrophilen Fasern, die als Trägersubstanz dafür vorliegen. Innerhalb einer Umhüllung der inneren Lage können zusätzliche Deckschichten aus anderen Stoffen wie Zellulose vorliegen, wobei dieses Produkt aus superabsorbierenden Substanzen, hydrophilen Fasern, Zellulose-Deckschicht oder Deckschicht aus hydrophilen Fasern und Umhüllung in einer zweiten Umhüllung vorliegt, die wenigstens einen der genannten Stoffe zusätzlich enthält. Auch dieser kann in dieser Umhüllung allein oder benachbart mit weiteren Stoffen in einer zusätzlichen Umhüllung vorliegen, so dass im Gesamtprodukt ein Innenraum und zwei oder mehr Umhüllungen vorliegen. Zwischen diesen können Zwischenlagen angeordnet sein, die wasserdicht, wasserdampfdicht, luftdurchlässig, semipermeabel oder anderweitig gestaltet sein können.

Eine Möglichkeit der Sicherstellung der Formstabilität liegt in der festen Pressung der vorgenannten Substanzen des Absorptionskörpers, bei der nicht notwendigerweise Klebstoffe benötigt werden.

So entsteht ein Produkt, das Stoffe wie CMC, hydrophile Fasern, Alginate oder andere der genannten Stoffe in umhüllter Form aufweist, mit dem Zweck, dass die osmotisch eher wirksamen Stoffe, wie granulatförmige superabsorbierende Substanzen, andere Stoffe des Verbandes rücktrocknen, und daher die Lebensdauer dieser Stoffe und deren Funktionsweise verlängert und daher Kosten, Material und Pflegezeit einspart. Parallel werden Phasen der Wundruhe verlängert, stabile Temperaturverhältnisse in der Wunde, die für Reparationsprozesse von Bedeutung ist, erreicht, und es kommt zu einer bilateralen Interaktion der Flachseiten der Stoffe. Denn einerseits wirken diese Stoffe in bekannter Form begünstigend in Richtung der Wunde auf die Wundregion ein, während sie an ihrer wundabgewandten Rückseite eine Rücktrocknung durch andere Anteile des Verbandsstoffes erfahren. Die näher zur Wunde applizierten Stoffe entfalten damit ihre Wirkung, und die gegenüberliegenden können eine Durchfluss-Spülung durch sich hindurch in die anderen Verbandsstoffanteile bewirken.

Die superabsorbierenden Substanzen, vornehmlich Granulate, können in Fasermatten eingepresst oder eingebracht sein, jedoch auch eingeklebt, geschweißt oder anderweitig fixiert sein. Hier können zwei Deckschichten einen Mix aus Cellulose und superabsorbierenden Substanzen überdecken, die beispielsweise als Airlaid-Matte hergestellt werden. Die superabsorbierenden Substanzen können aber auch in alle anderen genannten Stoffe eingearbeitet sein, insbesondere in Hydrokolloidfasern, Alginaten oder einer Vermischung verschiedener Substanzen der genannten Art oder mit dritten Stoffen.

Zwei Schichten aus Cellulose mit dazwischen liegenden superabsorbierenden Substanzen (ohne Cellulose) als Sandwich- Anordnung sind ebenfalls denkbar.

Da ein Verbandswechsel mit dem Absinken der Temperatur der Wundregion einhergeht, ist es denkbar, dem Absorptionskörper Reagentien zuzuführen, die im Sinne einer kontrollierten, bioverträglichen, exothermen Reaktion die Wundtemperatur stabil halten. Chemische Reaktionen wie Prozesse auf der Basis von Pfeffer können Berücksichtigung finden, insbesondere daher, als dass diese auch einen zusätzlichen osmotischen Sog errichten werden.

Das Produkt kann Verwendung finden als Teil einer Kompressionstherapie, Verband als Teil einer Therapie bei Kompartmentsyndrom oder bei der Vakuumtherapie vermittels Unterdruck. Im letzteren Falle addieren sich die beiden Druckarten, subatmosphärische und osmotische, so dass eine sehr suffiziente Ödemtherapie entsteht.

Bekannte Verbände mit Superabsorber-Teilchen erreichen die notwendige Potenz aus mehreren Gründen nicht. Die bekannten Verbände enthalten zu wenig superabsorbierende Substanzen, wobei die letzteren noch künstlich in ihrer Potenz geschwächt werden, da sie stark beabstandet von der Wundregion hinter Polyurethanschäumen oder Celluloselagen liegen. Bei anderen Produkten wird der osmotische Effekt mit Lösungen abgeschwächt. Insgesamt sind die derart applizierten Superabsorber-Teilchen von nur sehr geringer osmotischer Potenz, und ihr Potential wird innerhalb des Verbandes verbraucht.

Der Absorptionskörper umfassend die erfindungsgemäße Mischung soll Verwendung finden bei akuten und chronischen Wunden, iatrogenen Durchtrennungsstellen der Haut, Verbrennungswunden, nässenden entzündlichen Prozessen der Haut oder exulzerierenden Prozessen neoplastischer Genese, nässenden Infektionen, Fisteln, postoperativen Drainagen, Stomata, atopisch veränderlichen Arealen der Haut, gelenknahen Hautumschlagsfalten wie Achsel- oder Leistenhaut, Schleimhautoberflächen des Menschen und des Tieres sowie in Verbindung mit anderen Verbandstoffen, die lokaltherapeutischen Effekt haben und bei anderen Anwendungen, bei denen eine atraumatische Wundauflage indiziert ist. Spalthautentnahmestellen, plastische Deckungen, Abszesse und urologische Anwendungen seien gleichermaßen als Indikation fixiert wie proktologische Anwendungen und prophylaktischer Einsatz zur Prävention, z.B. für Eindämmung von Keimverschleppung und Reduktion der Ausbreitung von Keimen. Insbesondere steht die funktionelle Kombination mit den anderen breit beschriebenen Vorzügen im Vordergrund. Anwendungen unter atmosphärischem Unterdruck, seien sie kontinuierlich oder intervallgeschaltet, elektrisch oder manuell, Anwendungen bei Kompressionstherapie oder Kohlendioxid-Bad bilden weitere wesentliche Möglichkeiten.

Zum allgemeinen Verständnis des Begriffes "osmotisch aktiv" ist zu bemerken:
Gemeint sind Eigenschaften oder Summen aus physikalischen oder/und chemischen Prozessen, wie beispielsweise Elektronegativitäten, Molmassen, Ladungsausgleich, molekulare Interaktionen oder Verdünnungsneigungen von Stoffen wie Salzen, Zuckern und Proteinen, die aufgrund eigener Eigenschaften oder Mengen und Konzentrationen einen Druckgradienten im Sinne einer Strömungs- oder Einsogneigung von z. B. wässrigen Stoffen bewirken.

Das Vorhandensein einer semipermeablen Membran ist zwar für die experimentelle Demonstration notwendig, die zugrunde liegenden Gesetzmäßigkeiten finden auf molekularer Ebene aber auch ohne die Membran statt.

Gesetzmäßigkeiten aus den Bereichen und Themen Hypo-, Iso- und Hypertonie einzelner Komponenten, Diffusion, Angleichungsprozesse und Angleichungsenergien, Gleichgewichte, hydrostatischer Druck, osmotischer Druck, chemische Potentiale, Mischungseffekte, Konzentrationen, molare Dichte, Entropie und Gibbs-Energie können einzeln oder summenrechnerisch Anwendung finden. Alternativ oder ergänzend können Regelwerke der Prozesse von onkotischen Drücken, kolloidosmotisch relevanten Vorgängen und anderen Ereignissen, die von Anzahl und/ oder Beschaffenheit von Makromolekülen beeinflusst werden, vorliegen.

Verkürzt dargestellt, gelten hier vorzugsweise Prozesse, die zu Interaktionen mit Wasser führen, dieses lenken, speichern oder wenigstens kurzzeitige Verbindungen mit ihnen eingehen.

Die Bedeutung dieser Strömungsrichtungsdefinition hat weit reichende Konsequenzen. Beispielsweise sei genannt:
Es werden im Wesentlichen zwei Reinigungsprozesse an der Wunde durchgeführt. Zum einen entziehen die Superabsorber- Teilchen dem Wundgrund das Wundexsudat, wodurch eine physiologische Normohydratation (normaler Flüssigkeitsgehalt) unterstützt wird. Die Wundränder werden stabilisiert. Das Wundbett wird positiv konditioniert. Zum zweiten konzentrieren sich vor der Umhüllung Ausscheidungen mit geringem Wassergehalt, die an dem Umhüllungsmaterial anhaften und über den Wechsel des verbrauchten Absorptionskörpers entnommen werden können.

Der Absorptionskörper umfassend die erfindungsgemäße Mischung wirkt einer unerwünschten Granulation der Wunde entgegen. Da das pathologisches Exsudat aufgenommen wird, werden schädliche Proteasen aufgesaugt, zu denen beispielsweise MMPs (Matrix Metallo Proteasen) und TIMP's (Tissue Inhibiting Metallo Proteasen) zählen. Auf Grund aktiver Einsaugprozesse der schädlichen Proteasen wird eine sekundär antiinflammatorische Leistung erzielt. Der Absorptionskörper passt sich über den Saug- und Quellprozess an die Morphologie der Wundregion an.

Die mit der Wundflüssigkeit lokal durchtränkte und gequollene Matte füllt die jeweilige Wunde wenigstens teilweise aus.

Die Matte kann zunächst innerhalb der Umhüllung frei beweglich sein.

Idealerweise soll der aufgequollene Absorptionskörper ganzflächig an dem Wundbereich aufliegen. In diesem Zusammenhang kann die wundabgewandte Umhüllungswand aus einem Textil- bzw. Folienmaterial bestehen, das nicht oder nur wenig, während die andere, wundnahe Wand wesentlich mehr dehnbar ist.

Es wird eine möglichst große Oberfläche der im Kern verteilten Superabsorber-Teilchen sowie eine möglichst große Homogenität des Kerns angestrebt. Die in den Kern vorzugsweise klebstofffreien eingebetteten Superabsorber- Teilchen können eine im Wesentlichen gleichmäßige Matrix bilden. Dabei können die Superabsorber-Teilchen scharfkantig sein, damit ihre Oberflächen vergrößert sind. Die scharfkantigen Superabsorber-Teilchen verbessern zugleich die Haftung an den Textilfasern, die vorzugsweise zu einem wirren oder richtungsorientierten, mechanisch verfestigten Vlies verarbeitet sind. Die Textilfasern können geknickte, gebogene oder gefaltete Faserabschnitte von unterschiedlicher Breite und Länge sein. Die Textilfasern können wenigstens teilweise um die einzelnen Superabsorber- Teilchen herum verlaufen, so dass die Grenzflächen und somit die Saugkraft vergrößert werden können. Als Textilfasern eignen sich Polymer-Fasern und Naturfasern. Die Superabsorber-Teilchen können beispielsweise vernetzte, teilneutralisierte Akrylsäure-Polymere sein. Die Superabsorber-Teilchen können auch gelartig oder keramisch sein.

Den Superabsorber-Teilchen kann ein Kernvernetzer, vorliegend als Core-cross-linker (CXL) oder auch als Surface-Cross-Linker (SXL), oder eine Mischung beider Vernetzer zugesetzt sein.

Der Absorptionskörper wirkt mit derartigen Superabsorberteilchen als hydroaktiver Wundverband, der die aufgenommene Flüssigkeit wieder herausdampfen lässt.

Die Verwendungsmöglichkeiten des Absorptionskörpers können durch Zugabe von pharmakologischen Stoffen auf atomarer bzw. elementarer Basis, wie Verbindungen mit Zn, Ca, Na, erweitert werden. Zu den pharmakologischen Stoffen zählen beispielsweise Entzündungshemmer, Antibiotika, Wachstumsfaktoren, Homöopathika, Analgetika, Antipyretika und Desinfektionsmittel.

Der in der Umhüllung befindlichen inneren Lage und/oder der Umhüllung können Extrakte von Braunalgen, Carboxymethylcellulose, Alginate, Hydrokapillare, Hydrogele, Enzyme, Verbindungen auf der Basis von Keramik, Wachstumfaktoren, metallische Zugaben, beispielsweise auf der Basis von Silber, Gold, Platinum und Titan, weiterhin osmotisch aktive Substanzen, wie Salze, Zucker, Proteine, Enzyme, wie Peroxidase, zur Regulation des osmotischen Drucks zugeführt sein. Es kann die Zugabe von die Keimzahl reduzierenden Stoffen, wie Octenidin oder Polyhexamid vorgesehen sein.

Die pharmakologischen Stoffe können teilweise von körpereigenen Flüssigkeiten, wie pathologischem Wundexsudat, ohne den Einsatz von Elektrolytlösung aufgenommen und aufgelöst werden.

So wird über die Reduktion der Keimzahl durch physikalische Eigenschaften des Verbandstoffes die Gefahr von Mutationen, Biofilmen, Resistenzen und Infektionen verringert und die Wirksamkeit von antibiotischen Maßnahmen durch pharmakologische Eingriffe verbessert.

Es wird beispielsweise auf nicht-oberflächliches Exsudat ein in Richtung Absorptionskörper gerichteter Effekt erzielt, der die Förderung der Austrittsmenge sowie der Geschwindigkeit der Förderung verändert. Die pathologische Neigung zum besonders langsamen Fluss der Wundflüssigkeiten durch das Gewebe hindurch, die bis hin zur Stasis der Wundflüssigkeit in der Gewebetiefe führen kann, führt zu Zellödem und Überwässerung des Zellzwischenraums, so dass über den hier zu vollführenden Sog an äußerem Exsudat die Stasis in Richtung eines Flusses an Flüssigkeit zum Absorptionskörper hin bewegt wird. Die Überwässerung der Wundgrundtiefe wird damit reduziert, und ihre nutritive Gesamtsituation und damit ihr Heilungspotential wird verbessert.

Es werden im Wesentlichen zwei Reinigungsprozesse an der Wunde durchgeführt. Zum einen entziehen die Superabsorber-Teilchen dem Wundgrund das Wundexsudat, wodurch eine physiologische Normohydratation unterstützt wird. Die Wundränder werden stabilisiert, und es erfolgt auch eine gewünschte Wundbettkonditionierung. Zum zweiten konzentrieren sich vor der Umhüllung Ausscheidungen mit geringem Wassergehalt, die sich an dem Umhüllungsmaterial anhaften und über den Wechsel des verbrauchten Absorptionskörpers entnommen werden. Der Absorptionskörper umfassend die erfindungsgemäße Mischung wirkt einer überschiessenden Granulation der Wunde entgegen. Da das pathologisches Exsudat aufgenommen wird, werden schädliche Proteasen aufgesaugt, zu denen beispielsweise MMP' s (Matrix Metallo Proteasen) und TIMP' s (Tissue Inhibiting Metallo Proteasen) zählen. Auf Grund aktiver Einsaugprozesse der schädlichen Proteasen wird eine sekundär antiinflammatorische Leistung erzielt. Der Absorptionskörper passt sich über den Saug- und Quellprozess an die Morphologie der Wundregion an.

Über den Absorptionsprozess kann der Absorptionskörper so schwer werden, dass er überschießende Granulation durch sein Eigengewicht bremst und so der Wundbetthomogenisierung zuarbeitet. Das gewichtsbestimmende Element ist das Wundexsudat selbst.

So kommt es zu einem Verbandstoff, der bei flächenhaftem Anblick eine homogene Oberfläche hat, jedoch aufgrund seiner technischen Konzeption eine wundphasenspezifische Generalantwort bietet.

Hinzu kommt, dass nekrotische Bereiche der Wunde durch den Verbandstoff abgescheuert und abgelöst werden, wobei, die darauf folgende Nässung durch den Verbandstoff aufgefangen wird.

Wo eine Wunde infiziert ist, dort saugt der Absorptionskörper die Keime ab und entzieht der Wunde die Keime, die Toxine, das Exsudat, die Quellen der Entzündung und das Ödem. Da das Ödem und Teile der Entzündungsreaktion die Entstehung von organisierten Keimsystemen, Biofilm genannt, unterstützen, verkürzt der Absorptionskörper die Dauer der Infektion, reduziert die Wahrscheinlichkeit des Auftretens der Infektion und wirkt synergistisch weiteren antiinfektiven Maßnahmen zu. Die Ausprägung oder die Auf Keime wie MRSA (methizillin resistenter Staphylococcus aureus) werden bekämpft. Insbesondere im ambulanten, häuslichen, nicht-stationären Bereich führt die Eradikation dieser populationsgefährdenden Keime zu einer schnellen Abheilung chronischer Wunden, da nur hier sie ihre pathogenen Bedingungen vorfinden und zu ggf. lebensbedrohlichen, oft unbeherrschbaren Infektionen führen. Dieses gilt auch für Keime wie VRE (Vancomycin resistente Enterococcen) und CA-MRSA (Community acquired MRSA).

Wo eine Wunde sich in der Reinigungsphase befindet, nimmt der Absorptionskörper die ausgespülten Flüssigkeiten aktiv auf und bindet sie fest. Wo eine Wunde granuliert und sich neue Zellen bilden, schützt der Absorptionskörper sie vor der Zusammensetzung des pathologischen Exsudates der Wundregion der Nachbarschaft.

Wo eine Wunde hypergranuliert, bremst der Absorptionskörper sie in das erwünschte Wachstumstempo durch das Eigengewicht und das Gewicht der aufgenommenen Exsudation. Der Wundrand wird frei von entzündlichen ödematösen Prozessen der gesunden Haut gehalten.

Sogar dort, wo gesundes Gewebe ist, trägt die geringe Dicke des Absorptionskörpers unter Kompressionsverbänden dazu bei, dass Übergangskanten des Verbandes sich nicht sehr tief in die Haut des Patienten drücken, während viele andere Produkte eher dicker sind und Ab- oder Einschnürungen, Ab- oder Eindrücke oder nachhaltige Druckkanten mit der Gefahr von Perfusionsminderungen entstehen und dadurch Hautschädigungen fördern.

Kompressionsleistungen von umgebenden Kompressionsverbänden werden ungehindert weitergeleitet, während dem La Place'schen Gesetz folgend dickere Verbände bei gleiche Kompressionsumgebungen bei sodann höherem Radius die Kompression in der Tiefe behindern und weniger oder gar unkontrolliert weniger Kompression weiterleiten. Dieses ist insbesondere bei Schaumverbänden und dicken Zelluloselagen der Fall, da hier deren Elastizität diese Reduktion noch verschlimmern.

Der Absorptionskörper umfassend die erfindungsgemäße Mischung kann auch zur Feuchttherapie verwendet werden, ohne von vornherein nass zu sein.

Es kann an ausgewählter Stelle des Absorptionskörpers bzw. der inneren Lage eine signifikant höhere Menge an vergleichsweise kleinen, schnell saugenden Superabsorber-Teilchen vorliegen als an anderen, so dass lokal die Funktion eines Wundfüllers im Laufe des Absorptionsprozesses entsteht. Wird eine Wundfüllungsfunktion angestrebt, so bietet sich eine Umhüllung mit Wänden von unterschiedlicher Dehnbarkeit an. Insbesondere kann die wundnahe Wand eine größere Dehnbarkeit als die der gegenüber liegenden, wundabgewandten Wand aufweisen.

Ferner kann der Absorptionskörper von wenigstens einer Schaumstoff-Schicht unterlegt sein, die über wenigstens einen Klebepunkt oder eine periphere Naht mit der Umhüllung verbunden ist. Die Schaumstoff-Schicht kann offen- oder geschlossenporig, hydrophil oder hydrophob sein. Wird ein geschlossenporiger Schaumstoff eingesetzt, so empfiehlt es sich, in dem Schaumstoff durchgehende Öffnungen einzubringen.

Übrigens können die durchgehenden Öffnungen bzw. Löcher an jedem flächigen Material eingebracht sein, das Bestandteil des Absorptionskörpers ist.

Eine mögliche Zugabe von Tensiden führt zu einer signifikanten Erhöhung der Exsudation einer Wunde und kann wünschenswert sein. Hier sollten insbesondere biokompatible und systemisch wie lokal unbedenkliche Stoffe Einsatz finden, um die komplexen Kaskaden von Interaktionen von Drucksituationen, Perfusion, venösem Abtransport, Zellstoffwechsel, Motilität und Inertheit von Zellen wie Erythrozyten und Granulozyten, von körpereigenen oder zugegebenen Wachstumsfaktoren, immunrelevanten Zellen und Stoffen oder anderen für das Wachstum von Gewebe bedeutsamen Faktoren nicht zu stören. Eine mögliche Vasodilatation in der Wundregion kann hier begünstigend wirken, auch wenn sie Folge der Zugabe von Tensiden ist, da hier die Exsudation der Wundregion im Vordergrund steht und es zu einer Tiefenreinigung der Wundregion kommen soll. Dieses betrifft auch den optisch erkennbaren Wundboden, den kausaltherapeutisch relevanten Wundgrund sowie den Wundrand, Gewebeteile, die vor Exsudation und schädlichen Stoffen geschützt werden, indem die superabsorbierenden Granulate oder andere Wundkontaktflächen, wie Schäume aus PU, PE oder anderen Polymeren, Zellulose, Alginate, Hydrogele, Kohleverbindungen, Silberpräparate, Honig, Vliessotoffe, Nonwovens, Antiseptika, Träger dieser Stoffe, Folien oder fettimprägnierte Oberflächen diese Funktion erfüllen.

Diese Zugaben, wie die Tenside, oder andere genannte Stoffe können kovalent, lösbar oder frei an anderen Oberflächen verbunden sein.

Teile der Komponenten können unterschiedliche Lochstrukturen aufweisen, die die Interaktion von übergeordneten Lagen erleichtern. Der Durchtritt in die nächstgelegenen Lagen in beide Richtungen, additiv zur und absaugend von der Wunde, wird damit erleichtert.

In Kombination mit einem Schaumverband in einer gemeinsamen zusätzlichen Umhüllung werden weitere Vorteile erzielbar. So werden zwei Formen von Wundruhe erreicht: Bei starker Exsudation wird die Seite, die in der Umhüllung flächenhaft den Absorptionskörper zeigt, zur Wunde appliziert, und über den erwünschten starken Saugeffekt entstresst der Absorptionskörper die Wundumgebung, indem er ihr das unerwünschte und mit schädlichen Boten- und Inhaltsstoffen versehene pathologische Exsudat entzieht. Interstitielle und interzellulare, aber auch zelluläre und auch vaskuläre Räume erreichen die Nähe einer physiologischen Bewässerung, so dass Perfusion, arterieller Zustrom, venöser Abtransport und transmembranöse Diffusion optimiert werden und nicht durch lange Diffusionswege, durch pathologische wässrige Lösungen und reaktive biorelevante Enzyme behindert werden. Zellwachstum benötigt den arteriellen Anstrom von Proteinen, Sauerstoff wie auch den Einschuss von Gefäßen, Nerven und Funktionsträgern der Immunabwehr.

Bei schwächerer Exsudation kann die Schaumseite eines Schaumverbandes zur Wunde appliziert werden. Hier erzielt der Absorptionskörper einen mittelbaren Faktor der Wundruhe, indem er seine Saugkraft als Rücktrockner des Schaums nutzt. Hierzu ist es vonnöten, dass der Schaum annähernd durchfeuchtet ist, so dass z. B. durch Kapillarwirkungen der Durchstrom durch den Schaum gelingt und ein sanfter Einstrom in den Absorptionskörper entsteht. Hier trocknet der Absorptionskörper den Schaum zurück und bildet ein sekundäres Reservoir, das die Kapazität des Schaums um die Kapazität des Absorptionskörpers erhöht, ohne dass dieser nennenswerten Kontakt zur Wunde selbst hat.

Eine Umhüllung, die in dieser Ausführungsform den Schaum und den Absorptionskörper umgibt, kann aus perforierter PE-Folie gebildet sein, die kraterförmige Löcher gleicher oder unterschiedlicher Geometrie aufweist.

Das Prinzip des einzelnen Absorptionskörpers ermöglicht es, eine optische, vereinfachte Kontrolle des austretenden Wundexsudats bei Verwendung von derartigen, mit Superabsorber-Teilchen durchsetzten Absorptionskörpern durchzuführen, vorausgesetzt, dass der Absorptionskörper in Form einer entsprechenden, an die Wunde angepassten Größe eingesetzt wird.

Von großem Vorteil ist, dass das aufgesogene Wundexsudat eine begrenzte Position im Absorptionskörper einnehmen und behalten kann, wodurch die wundbenachbarte Umgebungshaut vom Wundexsudat nicht angegriffen wird.

Zur Unterstützung des allgemeinen Verständnisses der Funktionsweise lässt sich noch betonen:
Eine Überwässerung stellt beispielsweise ein Ödem dar, das auf dem Boden einer CVI (chronisch venösen Insuffizienz) entsteht. Die Gefäßwand der betreffenden Vene wird aufgrund pathologischer Prozesse durchlässiger, und nennenswerte Mengen an zunächst in der Vene befindlichen wässrigen Bestandteilen dringen nach außen in den Raum außerhalb der Vene. Dieses geschieht insbesondere in der Höhe des Knöchels am Sprunggelenk des Beines, da hier die Blutsäule der Vene ihren Beginn findet und hier der hydrostatische Druck am größten ist.

Es kommt zu einer Überwässerung der Umgebung, der gesunden Zellen, der Haut und der Zellenzwischenräume. Diffusion arterieller und nutritiv bedeutsamer Stoffe wie auch der Abtransport verbrauchter Stoffe über die Vene werden eingeschränkt. Die Zellen gelangen in eine reduzierte Stoffwechsellage, biorelevante Prozesse und fein orchestrierte Stoffwechselkaskaden gehen zu Grunde und die Zellen sterben ab. Ein venöses Ulcus cruris entsteht, bricht bis zur Haut durch und bleibt zeitlebens überwässert.

Derselbe Prozess in der Lunge würde über die Steigerung der Nierenleistung bekämpft, zumindest dann, wenn die ganze Lunge betroffen wäre.

Die Bekämpfung dieser Überwässerung am Unterschenkel mit Hilfe von Diuretika ist nicht angezeigt, da eine Vielzahl von medizinischen Gründen dagegen spricht. Hier entstünde ein Eingriff in kardiorelevante Systemkomplexe, und da eine akute Lebensgefahr im Gegensatz zum Lungenödem nicht erkennbar ist, müssen andere Wege gefunden werden.

Im Gegensatz zu diesen Erkenntnissen sieht die Praxis der Therapie vor, Verbände vergleichsweise niedriger Saugkraft und mangelhafter Retention zu applizieren. Das Ödem im Beingewebe wird dabei ungerichtet, passiv, lokal und physikalisch bekämpft.

Derartige Therapieansätze haben gemeinsam die Kombination aus Kausaltherapie (Herzmedikation zur Herzkräftigung und daher Bekämpfung der Blutstauung in der Lunge einerseits sowie Tragen von Kompressionsstrümpfen zur Bekämpfung des Austritts von venösem Presswasser andererseits) und Symptombehandlung (Diurese für Entwässerung der Lunge einerseits wie auch die Auflage angeblich saugstarker Verbände andererseits).

Während die Funktionseinschränkung von z.B. Lungengewebe als bedrohlich eingestuft wird, bleibt die ödematöse Aufquellung von gelenksnahem Gewebe als vergänglich und damit unproblematisch eingeschätzt. Hier entsteht das erste Ungleichgewicht, das zwar mit Blick auf Vitalfunktionen und Lebenserhalt nachvollziehbar ist, jedoch den kurzen Weg in patientenseitiges Leid, Schmerzen, chronische Erkrankungen, Kosten, manchmal auch Verlust des Arbeitsplatzes und eine Vielzahl weiterer Nachteile unterschätzt.

Jeder Mediziner weiß, dass verordnete Kompressionsstrümpfen oft nicht sinngemäß und konsequent getragen werden. Hier zeigt sich, dass die wesentliche Funktion, Beingewebe zusammenzupressen, nicht zum Alltag der Patienten passt, da das Anziehen, wenn überhaupt möglich, sehr mühsam ist und als sehr unangenehm empfunden wird. Im Sommer schwitzen die Patienten, und das Ausziehen ist auch oft schwierig.

Im Ergebnis wird entweder eine Kompressionsstrumpfhose verordnet, die zu wenig Druck erzeugt, die aber wenigstens getragen wird, oder es werden gar keine Strümpfe getragen. Der kausaltherapeutische Ansatz, der für die Lunge in der medikamentösen Steigerung der Herzleistung liegt, versagt oft bei der venösen Insuffizienz, denn diese ist nur über gute Kompressionstherapie erzielbar. Findet diese nicht oder nicht ideal statt, so wird die Vene dauerhaft weiter nässen.

Die Therapie mit Unterdrucksystemen, die sogenannte Vakuum- Therapie, scheint wirksamer zu sein, da diese über subatmosphärische Druckverhältnisse an der Tiefe der Austrittsstelle von Flüssigkeiten zieht.

Da diese Therapiewahl jedoch auch eine Vielzahl von Nachteilen hat, die im Spektrum von hohen Kosten und Immobilität über hohe Anforderungen zur Anwendung bis hin zu Todesfällen reichen, gilt es, neben den nephrologisch-diuretischen und neben den vakuumunterstützten Therapieansätze einen dritten Ansatz zu finden.

Vorteilig wäre hierzu ein einfach anzuwendender Verband, der sich bekannte physikalische Verhältnisse und Gegebenheiten zunutze macht und auf pharmakologische Prozesse verzichtet.

Vorliegend wird ein Verband umfassend die erfindungsgemäße Mischung gewählt, der die Vorteile der subatmosphärischen Ansätze erzielt, ohne mit den Nachteilen von Unterdruck der Luft, wie etwa die Erschaffung von luft- und sogdichten Kammern, kämpfen zu müssen.

Dieses gelingt über den Einsatz von signifikanten osmotischen Unterdruckverhältnissen, die in Form eines Verbandes flächenhaft auf die Wundoberfläche gelegt werden und sich in neuartiger Form physikalischen Gesetzmäßigkeiten anschließen.

Vorzugsweise weist die innere Lage, die die Form einer Matte an Zellulose Trägermaterial für Superabsorber-Partikel hat, eine Flächenmasse von mehr als 300 g/m² und hat mehr als 50% osmotisch wirksame Substanzen. Die Bindungskraft liegt bei 0,5 bis 2g NaCl in 0,9%iger Lösung pro g der inneren Lage.

So wird über den angestrebten Verband eine derart hohe osmotische Potenz (erste Kraft) in die Wunde gelegt, dass Flüssigkeiten der Wundoberfläche unverzüglich eingesogen werden. Deren Kohäsionskräfte (zweite Kraft) übertragen diese Flußrichtungsweisung auf benachbarte Wundflüssigkeiten, so dass eine Tiefenwirkung entsteht. In der Wundtiefe wird daher eine Strömungsrichtung der überschüssigen Wundflüssigkeiten definiert, und aufgrund mehrerer Einflüsse wird diese erhalten und fortgeführt.

Hierzu ist neben der Kohäsionsneigung von Flüssigkeiten auch deren Adhäsionsneigung (dritte Kraft) zu nennen, so dass zwar nur wenig Flüssigkeit den direkten osmotischen Sog erfährt, dessen Richtungsweisung jedoch auch in der Tiefe angelangt. Es ist zu vermuten, dass auch in der Tiefe des Gewebes ein Mechanismus (vierte Kraft), der auf dem Boden von Kapillaraszension entsteht, zwischen den Zellen stattfindet, so dass hierdurch die Erhaltung der Flussrichtungsdefinition unterstützt wird.

Der in der Tiefe befindliche Pressdruck (fünfte Kraft) der durchlässigen Vene unterstützt diesen Effekt, da sie nur den Ausweg der Flüssigkeiten nach peripher und damit zur Haut kennt. Hier wäre die fünfte Einflussgröße beschrieben, die angestoßen durch den hohen osmotischen Sog zu einer Entlastung der Überwässerung des Wundgrundes führt.

Die Summe dieser Faktoren führt zu der sechsten Größe. Diese ist bedeutsam für das Funktionieren des Zusammenspiels der ersten fünf.

Hierzu muss etwas ausgeholt werden, denn physikalische Gesetzmäßigkeiten zum Zusammenhang mit der Strömung sind hier für die Bemessung der herrschenden Kräfte von Bedeutung:
Als Ursache einer Strömung betrachtet man aus physikalischer Sicht immer eine Druckdifferenz Ap auf einer Strecke eines Rohrsystems. Zwar liegt in unserem Falle kein Rohrsystem im engeren Sinne vor, jedoch kann das zu durchströmende Gewebe als geschlossenes System betrachtet werden, durch das sich das Wundsekret eigene Wege und Kanäle suchen muss. Hierbei unterliegt es bremsenden Einflüssen wie Scherkräften, Strömungswiderständen, Reibungsverhältnissen, mechanischen Druckdifferenzen, SchubSpannungen, Stromstärken, wechselnden Durchmessern der Wege und Kanäle und anderen Einflußgrößen. Trotz ihrer langsamen Strömung liegen die Bedingungen für die Kennzeichnung als laminare Strömung nicht vor; hingegen ist die Bedingung der höheren Flussgeschwindigkeit zur Kennzeichnung als turbulente Strömung auch nicht gegeben.

In der Annahme, dass hierzu die Reynoldsche Zahl Re, eine empirische Zahl, die etwa ein Verhältnis zwischen Impulsen (Kraftstöße) und durch Reibung mögliche Abfederung beschreibt, unter der Grenze der Umschlagshöhe von 2300 liegt, läge es nahe zu vermuten, dass eher die Bedingungen einer laminaren Strömung bei unbehandelten Ulcera der beschriebenen Genese vorliegen.

Konstante Gesetzmäßigkeiten zur Flussrichtung und zur Flussgeschwindigkeit liegen sicherlich nicht vor, vielmehr finden sich Vermischungen von verschiedenen Gesetzmäßigkeiten wie das Gesetz von Hagen-Poiseuille oder das Gesetz von Bernoulli oder des Einfluss des Fahraeus-Lindquist-Effekt (Einfluss des Gefäßdurchmessers, Axialmigration).

Im Anschluss an diese eher rein physikalische Betrachtung von Fließverhältnissen ist von Bedeutung, dass in der unbehandelten Wundregion unterschiedlichste Phasen, Bedingungen, Drücke und Flussraten bestehen.

Hieran anschließend kommen die obigen fünf Kräfte zusammen. Denn das osmotische Potential eines Superabsorbens durchbricht diese Unordnung und erschafft eine Fließrichtung der Wundflüssigkeit, indem er an der Oberfläche saugt und wundtiefe Flüssigkeiten über die Summe der genannten Kräfte mit abgesaugt werden. Der Impuls an den ersten Wassermolekülen in den Verband hinein generiert einen Impuls für alle nächst folgenden Wassermolekülen, indem sie über die genannten Kräfte oder auch über "van der Waals - Kräfte" kettenförmig miteinander verhaftet sind. Der Zug am ersten Glied erschafft einen Zug am letzten Glied, und im Sinne einer selbständigen, dynamischen Fortsetzung dieser Prozesse folgen die Wassermoleküle dem einmal eingesetzten Strom dauerhaft, da das osmotische Gefälle des Superabsorbens eine Einbahnstrasse in das Produkt hinein erschafft.

Das Gefälle an osmotischer Kraft und Kohäsion lässt eine Entwässerung in der Gewebetiefe, direkt im räumlichen Umfeld der insuffizienten Vene entstehen. Der auf die Wundoberfläche aufgelegte Verband hat seine primäre Funktionserfüllung in gerade dieser Gewebetiefe und entzieht dort das Wasser und dehydriert das Ödem. Hierzu wirkt er durch das zwischen Wundboden und Wundgrund gelegene Gewebe hindurch und saugt transtissual perivenöse Überwässerungen ab, ohne mit diesen in besonderer räumlicher Nähe zu stehen. Das hohe osmotische Potential entzieht der Wunde ihre nicht-oberflächlichen Ödeme. Sanft und körperschonend, und insbesondere ohne Zellen oder Luft zu dehydrieren, reguliert das Ausmaß des Durchflusses von Exsudat durch diese Kanäle die Saugkraft, indem bei hohen Mengen an Exsudat das Produkt aktiv anspringt und sich der Wundbodenoberfläche noch eher zuwendet, hingegen bei niedrigen Mengen nur Luftfeuchte über Verdampfungsprozesse erschaffen wird. In allen Fällen werden auch die Prinzipien der Feucht-Therapie gewährt und unterstützt.

So entsteht ein Saugverband, der im Verständnis der unter hohem osmotischem Druck sanft biorelevante Noxen wie Keime, Toxine, Proteasen etc. aktiv entfernt. Hierbei ist das pathologische Exsudat weniger als beherrschungswürdiges und wundheilungsschädliches Problem anzusehen, sondern es wird im Gegenteil zum heilungsförderlichen Gegenstand: Es bildet das Transportmedium für diesen reinigenden Saugeffekt, und wird nach Eintritt in den Verband als Luftfeuchte wieder an die Wunde abgegeben, nachdem es von seinen schädigenden Substanzen befreit wurde, da diese nicht mit verdampfen, sondern im Verband zurückbleiben. Der Verband reinigt damit Teile des Exsudates, nachdem dieses eine eigenen und andere Schädlinge in den Verband hineingetragen hat.

Den Weg in den Verband hinein hat es gefunden, weil es angeregt wurde, in eine vordefinierte Richtung zu fließen. Diese Dynamisierung von eher statischem Wasser in nur chirurgisch zugänglicher Region und in den Zellzwischenräumen führt zur Ausbildung von Flusskanälen und damit zur Entlastung des perivenösen Gewebes. Im Laufe dieses Prozesses wird das pathologische Exsudat zur Spüllösung der Wundoberfläche und der Wundtiefe und hält durch den kontinuierlichen Strom die Flusskanäle offen.

Im Unterschied zur Vakuum-Therapie wird die maximale Saugleistung über die Dauer der Anwendung lokal und sukzessiv reduziert und ist daher nicht derart statisch wie ein elektronisch eingestellter Pumpendruck und damit auch weniger gefährlich. Die erforderliche Saugkraft des Verbandes, die über die Superabsorber erreicht wird, wird von dem Ausmaß der flächenanteilig vorhandenen Exsudation reduziert und damit bestimmt, so dass hier von einer wundadaptierten Saugkraftregelung gesprochen werden kann. Abhängig von der Wundheilungsphase und der Bedingung jedes Quadratzentimeters einer Wunde entsteht eine Saugleistung, die typisch ist für die jeweilige Wundsituation.

Dazu gesellt sich der Umstand, dass im Gegensatz zur Vakuum- Therapie der Verband die ausgespülten Keime und das möglicherweise infektiöse Exsudat direkt nach Austritt aus der Körpertiefe eliminiert und die Keime unmittelbar nach Einstrom in den Verband bekämpft. Das Exsudat wird nicht abgeleitet und gefährdet damit nicht das Personal. In Einzelfällen können endemisch oder epidemisch relevante Keime nur als teures und kostenintensives infektiöses Flüssiggut entsorgt werden.

Hierzu ist von Bedeutung, dass der gewünschte Effekt eines Druckes von wenigstens etwa 20 mm Hg bedarf, da unterhalb dieser Werte der Sog in den meisten Fällen nicht ausreicht. Die unmittelbare Nähe des Trägers des osmotischen Potentials, die über die dünne Umhüllung zum Wundboden erschaffen wird, bringt dieser Unterdruck auf.

Dieses lässt auch einen anderen signifikanten Unterschied zu anderen Verbandstoffen deutlich werden. Denn wenn diese, insbesondere Zellwatte, Fluffpulp oder Vliesstoff enthaltenden Verbände mit Exsudaten in Kontakt kommen, verlieren sie unmittelbar ihre strukturelle Integrität und degenerieren bei Durchfeuchtung. Bereits sehr bald nach Kontaktaufnahme mit Exsudat ist dieser Verband nass und hat keine Saug- und Zugkraft mehr. Vor dem Hintergrund der Notwendigkeit, in der Gewebetiefe abzusaugen, stellt sich Eignung eines solchen Verbandes als Verbandmaterial für ein venöses Ulcus cruris in Frage. Wenn auch derartige Erkrankungen mit modernen, hydroaktiven Verbänden wie Polyurethanschäumen behandelt werden, lässt sich erkennen, dass hier noch kein ausreichender Schritt nach vorn, getan wurde.

Exsudat und Keime bilden die Sollbruchstelle der Heilung. Exsudat unterhält Keime, und Keime führen zu Exsudat. Ödeme führen zu verminderter Immunantwort, und der Kreislauf schließt sich. Durchbricht man diesen Lauf, heilt und schließt die Wunde.

Dieser Gedanke berücksichtigt insbesondere, dass Wunden nicht an jeder Stelle homogen und gleich sind. Viele Wunden erleben alle Phasen der Wundheilung zeitgleich, indem sie hier nekrotisch und abgestorben, dort infiziert und belegt, hier exsudierend und wässrig, dort fibrinös und hier wieder hypergranulierend sind. Hier ist der Wundrand, dort eine Entzündung, hier frisch epithelisiertes Gewebe und dort eine blutende Läsion durch die schmerzhafte Entfernung verklebter Verbände.

Die selbständige Adaptation an die jeweiligen Bedingungen gelingt über den hier beschriebenen Verband, da er jeder der genannten Wundsituationen entspricht, indem er an jeder Stelle die passende Gegenantwort liefert. Nekrotische Regionen weicht er über Luftfeuchte auf und scheuert an ihnen, Keime saugt er über das Exsudat der regionalen Entzündung mit auf, Beläge konzentrieren sich an der Umhüllungsaußenwand auf und werden mit der Entfernung des Verbandes mit entfernt, wässrige Exsudation wird mit hoher Rückhaltekraft entzogen, Hypergranulation bremst der Verband durch das Eigengewicht und das Gewicht der aufgenommenen Exsudation, frisches Epithel schützt er vor Exsudat und Verklebungen finden nicht statt, und wenn doch, dann kann ein handelsübliches Wunddistanzgitter als Primärverband verwandt werden.

Alle typischen Vorteile werden noch optimiert, indem der Verband vollflächig Kontakt hat zur Wunde. Hier gilt es zu berücksichtigen, dass die Oberfläche einer Wunde nicht glatt wie eine Glasscheibe ist, sondern im Gegenteil eine extrem inhomogene Morphologie aufweist. Aus Perspektive einer kleinen Zelle ist der Vergleich mit einer Luftaufnahme Deutschlands durchaus angemessen: Hier im Süden haben wir Berge, dort oben eher flaches Areal, hier in Ballungsgebieten stehen viele hohe Gebäude und hier liegen viele Seen und vielleicht sogar auch einmal etwas unter Normalnull. Die Erkenntnis, dass kaum eine Wunde nur eine einzige Heilungsphase und Heilungssituation aufweist, führt zu der Notwendigkeit eines Verbandes, der mehrere Phasen zeitgleich angemessen behandelt. Dieses sei durch den gewählten technischen Ansatz gewährleistet.

Es kann empfehlenswert sein, als vollflächige primäre Kontaktschicht einen der genannten Schaumverbände zu nutzen, sofern die Vorzüge des Absorptionskörpers zusätzliche Verwendung finden. Dieses könnte dadurch geschehen, dass der Schaum Wundkontakt hat und der Absorptionskörper ihm rückseitig direkt aufliegt, um zum einen die Angleichung an eine sehr inhomogene Wundbodenmorphologie über einen Schaum sicherzustellen, der als Durchfluss- und Kontaktkörper die Wundflüssigkeiten direkt in den Absorptionskörper leitet. Auch die Erfüllung dieser Funktion über Alginate, Kohleverbände oder Baumwoll- und Vliesstoffe ist denkbar.

Werden Polyurethanschäume verwandt, so neigen diese dazu, sich an den Rändern aufzurollen. Die Verwendung des Absorptionskörpers rückseitig bildet hier einen erwünschten mechanischen Gegendruck und trägt so zur Beibehaltung der vollflächigen Kontaktfläche bei.

Die äußere Form des Verbandes kann in Form von Quadraten oder anderen Geometrien vorliegen, kann aber auch anatomische Formen haben wie Handschuhe zur Behandlung von Extremitäten beispielsweise.

Von zentraler Bedeutung ist in allen Fällen, dass ein neues Verständnis von Wundbehandlung, Ödemtherapie und Gefäßinsuffizienzbehandlung entsteht, indem venöses Presswasser, Ödeme und deren Flüssigkeiten dynamisiert und zur Hautoberfläche gelenkt werden, um dort kraftvoll entzogen zu werden, so dass die Wunde sich verschließen kann. Entstressung, Entquellung, Entflutung und Entzug schädlicher Stoffe führen zu Befriedung, Tiefenentlastung und Entstauung von Gewebe, das über diesen Spülprozess vom tiefen perivenösen Gewebe bis hin zum Wundboden gereinigt wird.

Dieses neue Verständnis beinhaltet auch die Möglichkeit, mit der Vakuum-Therapie kombinierbar zu sein, um dort eine Verstärkung der Sogleistung zu erreichen. Modulationen, die über eine Verringerung der Luft-Sogleistung ermöglicht werden, da osmotische Saugleistungen additiv dazukommen und daher Regulationen der synergistischen Faktoren erlauben, sind denkbar.

In allen Fällen, mit und ohne Vakuum, wird im Körper Exsudation und Verschmutzung, die über die Spülleistung in den Absorptionskörper hineingetragen wurden, gesammelt.

Synergistische Faktoren können sich auch gegenseitig bedingen, beispielsweise in den Fällen, wo z. B. über Tenside die Exsudation angeregt und gesteigert wird, ist der Einsatz des Absorptionskörpers nötig, um die hervorgerufene Exsudation zu beherrschen.

Andere Zusätze können auch Naturstoffe sein, beispielsweise Extrakte von Früchten oder Nüssen; genannt seien hier insbesondere die Saponine, Extrakte der Waschnüsse: Früchte des Waschnussbaums (Sapindus mukorossi).

Die Quellstoffe können über Ionentauschprozesse zu Reduzierungen der Keimzahl in der Wundregion führen, indem Depolarisationen und deren Fortleitung behindert werden oder Konzentrationsgradienten an der Zellmembran von Organismen oder Keimen reduziert werden. Hier kann die Bekämpfung von resistenten Keimen eine endemische oder epidemische Rolle spielen, denn die kolonisierte Wunde erhält oft den Keim und der Keim erhält oft die Wunde. Die Durchbrechung dessen ist eine nennenswerte Maßnahme bei der Eindämmung von Ausbreitungen der Keime und deren Infektionen.

Hier spielt die Möglichkeit, die superabsorbierenden Granulate in ein Bett aus geschnittenen und geknickten Cellulosefasern einzulagern, bevor sie quellen, eine nennenswerte Funktion bei der Erzeugung der Luftfeuchte in der Wundregion, da hierdurch die erzielte Oberflächenmaximierung gelingt. Deckschichten aus Cellulose, oberflächenstarke Granulate und oberflächenintensive Fasern führen in ihrer Summe zu viel Raum und viel Fläche für die Verdampfung aufgenommener flüssiger Anteile und damit für die Erzeugung und Aufrechterhaltung des feuchten, angestrebten Wundklimas.

Diese Form einer inneren Lage als Superabsorber-Träger kann insbesondere bei Airlaid-Matte vorliegen.

Nichtsystemische, lokale, aber notwendige Entwässerung, wird daher über einen Exsudat begierigen, mit hoher osmotischer Potenz versehenen Verband durchgeführt. Denn es ist nicht einsehbar, dass eine Vene krank ist, und allein deshalb Haut und Bindegewebe untergehen.

Denn nur über diese Spülprozesse ist es denkbar, Verunreinigungen, Rückstände von Keimen, Zelltrümmer, Stoffwechselprodukte von Bakterien und in der Wundregion befindlichen Zellen an die Oberfläche zu fördern, um sie dort direkt einzusaugen und kontrolliert zu entfernen.

Vom Vorteil ist, dass neben überschüssigen Wundflüssigkeiten auch darin enthaltene wundheilungsschädliche Substanzen mit entzogen werden und eine kürzere Verweildauer im Gewebe des Patienten aufweisen können. Die Folgen pathologischer Prozesse können reduziert werden.

Gemäß einer Ausführungsform weist die innere Lage mehr als 40% osmotisch wirksamer Substanzen auf, bezogen auf das gesamte Gewicht. Osmotisch wirksame Substanzen können auch in der Umhüllung vorliegen.

Die osmotisch wirksamen Substanzen können in Pulver- oder Granulatform vorliegen, wobei die Granulatform sowohl die regulären kugeligen bzw. prismatischen als auch unregelmäßigen Formen, wie eine Sandkornform, aufweisen kann.

Die innere Lage kann für die Aufnahme von Flüssigkeiten ausgelegt sein, deren spezifisches Gewicht etwa dem des Wassers, also einem Wert 1,00 entsprechen, oder das besagte spezifische Gewicht geringfügig überschreitet und bei etwa 1,020 liegt, wobei eine Auslegung für höher viskose Flüssigkeiten, deren spezifisches Gewicht beispielsweise einen Wert 3,00 erreichen kann, ebenfalls möglich ist.

Zu den osmotisch wirksamen Substanzen zählen vor allem sogenannte Superabsorber, allerdings kann die innere Lage zusätzlich andere osmotisch wirksame Substanzen, wie Salze, Zucker, Proteine, Elektrolyten etc., aufweisen.

Die vorgenannten osmotisch wirksamen Substanzen können auch Bestandteil der Umhüllung sein. Außerdem kann die Umhüllung antimikrobielle, geruchshemmende, desinfizierende, fungizide oder andere wundheilungsförderliche Stoffe, wie Pharmaka, aufweisen.

Die angegebene Konzentration der osmotisch wirksamen Substanzen unter Inanspruchnahme von Adhäsions- oder Kohäsionskräften von Wasser trägt zu einer Regulation von Überwässerungen in tieferem, nicht an der Wundoberfläche gelegenem gesundem oder pathologischem Gewebe bei. Die osmotisch wirksamen Druckgradienten werden im Wesentlichen über Ionenaustauschprozesse erreicht.

Die Überdimensionierung von Quellstoffen, darunter insbesondere von Superabsorber-Teilchen trägt dazu bei, dass das Wundexsudat, sowie Feststoffpartikeln und Keime aus dem Wundgrund mitgerissen werden können.

Der Zusammensetzung des Absorptionskörpers umfassend die erfindungsgemäße Mischung beeinflusst über Konzentrationsgradienten, -gefälle und -differenzen im Vergleich zum Wundgebiet die Flussrichtung von Wundflüssigkeiten. Der in Richtung Hautoberfläche wachsende, durch den Absorptionskörper erzeugte Sog kanalisiert die Wundflüssigkeiten, beschleunigt deren Transport und führt zur lokalen Fixierung von gewebe- und wachstumsschädigenden Proteasen und Faktoren an der inneren Lage und schützt auf diesem Wege effektiver das Wundgewebe, das Wundrandgewebe und die Wundregion.

Die Potenz des osmotisch wirksamen Stoffes kann derart gewählt werden, dass dem Ausmaß einer lokalen hydrostatischen Druckerhöhung aufgrund einer gesteigerten Permeabilität der Gefäße auftritt, ein wirksamer osmotischer Druckgradient gegenübersteht, der die Überwässerungssituation des Gewebes zwischen den beiden gegensätzlichen Zentren durch definierte Flussrichtung und Flussgeschwindigkeit reguliert, so dass funktionell die Summe aus hydrostatischem Überdruck und osmotischem Sog zu einer geringeren Ansammlung von Flüssigkeit im interstitiellen Raum führt. Die osmotische Potenz des Absorptionskörpers kann auch zelluläre Überwässerungen regulieren.

Die Potenz des osmotisch wirksamen Stoffes des Absorptionskörpers kann derart gewählt werden, dass dem Ausmaß einer lokalen kapillaren Hypertonie eine proportionale osmotische Sogwirkung gegenübersteht, die das Verbleiben von Flüssigkeit in unphysiologischer Menge in Richtung der physiologischen Hydratation verkürzt.

Weiterhin kann die innere Lage oder die Umhüllung als Depot für Medikamente oder Lösungen dienen, die an die Wundregion kontinuierlich abgegeben werden können.

Die innere Lage kann aus einer Vermengung von superabsorbierenden Polymeren mit Zellulose als Trägermaterial, sowie zwei flächenhaften Deckschichten bestehen. Dabei kann wenigstens eine der Deckschichten aus Zellulose bestehen, wobei sich die Raumdichte der Deckschicht von der des Trägermaterials unterscheiden kann.

Die innere Lage kann über Beimengungen verfügen, die auf Basis von Acrylsäure oder auf Basis von Aktivkohle hergestellt sind. Die Aufnahmekapazität des Absorptionskörpers kann oberhalb eines Wertes liegen, der etwa 95g/100cm³ des Absorptionskörpers beträgt, gemessen nach DIN EN ISO 53923.

Der Absorptionskörper kann an wenigstens einer Stelle Stärkepolymere enthalten, da diese einen besonders hydrophilen Charakter aufweisen. Als Stärkepolymere können beispielsweise native Stärkepolymere auf Basis von Mais, Kartoffeln oder Reis zum Einsatz kommen, die eine hohe Feuchtigkeitsempfindlichkeit besitzen.

Der Absorptionskörper kann kühlbar sein und wenigstens teilweise vorgesättigt mit Flüssigkeiten, in gefrorener Form auf akute Wunden, Nähte oder andere Hautareale auflegbar sein. Die Flüssigkeit können antimikrobiell wirksame Flüssigkeiten, Hyaluronsäure oder andere dem Wundheilungsprozess förderliche Substanzen, wie quaternäre Ammoniumsalze, sein.

Der Absorptionskörper kann auf Körperoberflächen auflegbar sein, die ein angelegtes elektrisches Feld aufweisen. Dabei wird insbesondere der Gleichstrom bevorzugt. Es können auch mikrobielle Stoffe, die infolge Fermentationsprozesse entstanden sind, zugeführt sein.

Einer der wirksamen Substanzen kann ein Stoff sein, der die Ausbildung von molekularen Netzwerken fördert. Dies kann insbesondere auf dem Boden von hydrophilen Prozessen entstehen, wenn Verknüpfungen mit Wassermolekülen entstehen und die speichernden Körper über Vernetzungen verfügen.

Die technischen Ansätze der hydrophilen Vorgänge können auf dem Boden von grundsätzlichen physikalischen Vorgängen, wie dem Streben nach Ausgleich und Harmonie, beruhen. Entropisch getriebene Prozesse, Enthalpie, Brown'sche Molekularbewegung wie auch die Ausnutzung von Verdünnungsneigungen können im Vordergrund stehen. Auch die Reduktion elektrostatischer Wechselwirkungen kann im Vordergrund stehen.

Der Absorptionskörper kann eine Substanz enthalten, die teilweise oder gänzlich von wenigstens teilweise flüssigkeitsdurchlässigen Ummantelungen aus Silikon umgeben ist. Dazu kann eine Matte von Airlaid-Typ vorliegen, die nicht reine Cellulose als Trägermaterial für Superabsorber- Partikel aufweist, sondern Carboxymethylcellulose (CMC). Hier unterliegt z.B. ein Gemisch von Superabsorber- Partikeln mit Carboxymethylcellulose einer möglichen Abdeckung mit einer dünnen Cellulose-Schicht; Diese Abdeckung kann an mehreren Flächen vorliegen oder ganz fehlen. Diese Ausführungsform ist besonders günstig, weil die in der klassischen Matte eingearbeitete Cellulose stark gepresst wird und als hart und verletzend empfunden werden kann, während die Carboxymethylcellulose-Fasern möglicherweise weicher bleiben. Anstelle oder vermischt mit diesen Fasern können weitere Stoffe wie Alginate oder andere hier genannte Substanzen zugefügt sein. Der Quellvorgang kann durch eventuelle Hüllen teilweise hindurch treten und direkten Kontakt zur Wundoberfläche haben.

Inhomogene Trägersubstanzen für Superabsorber-Partikel können in wenigstens einer Lage des Absorptionskörpers vorliegen, um der Lage bzw. der Matte nicht nur die Speicherungsfunktion zu geben, sondern darüber hinaus weitere Funktionen wie Temperaturerhalt, Wundbodenadaptation oder Keimreduktion durchzuführen. Hier können z.B. Silberpräparate oder Polyhexanid und Aktivkohle zusammentreffen. Die Polyhexanide können in getrockneter Form, flüssig oder gebunden an Teile des Absorptionskörpers vorliegen.

Der Absorptionskörper kann in Körperöffnungen, Falten, Erhebungen oder anderen Cavitäten des Körpers des Patienten einlegbar sein, wo er insbesondere zur Heilung von Geschwüren und Substanzdefekten an Gewebe, beispielsweise bei dentalchirurgischen Eingriffen in der Mundhöhle, eingesetzt werden kann.

Die innere Lage kann flächenmäßig 3% bis 90% kleiner als die Umhüllung sein, wobei sich um eine Fläche der Umhüllung handelt, die durch die umlaufende Naht begrenzt ist.

Vorzugsweise besteht die Umhüllung aus einem Gewebe oder Vlies, das wenigstens 20 Gramm pro m² wiegt. Die Umhüllung kann aus gewebten oder vliesartig zusammengesetzten Kunstfasern, wie Polypropylen- oder Polyethylenfaser, aus Naturfasern oder einer Mischung von Natur- und Kunstfasern gebildet sein. Die Umhüllung kann sowohl für flüssige als auch für visköse Stoffe durchlässig sein.

Die Umhüllung kann aus einem zusammengelegten oder aus zwei Blättern bestehen, die an ihrer Peripherie miteinander verbunden sind, wobei die Verbindung der Blätter miteinander durch Ultraschall, Verklebung, Vernähung oder ähnliche thermische, physikalische oder chemische Prozesse hergestellt sein. Weiterhin kann die Umhüllung an ihrer Peripherie weiche Kanten aufweisen, die beispielsweise durch Überschreitung der Nahtfläche mit Umhüllungsmaterial entstanden sind.

Die Umhüllung kann Poren bzw. Maschen aufweisen, die jeweils kleiner als die Ausmaße der zu absorbierenden Substanzen in Granulat- oder Pulverform sind. Die Poren bzw. Maschen können auch größer, vorzugsweise geringfügig größer als die Superabsorber-Teilchen sein, wenn die letzteren in das Trägermaterial der inneren Lage eingebettet bzw. dort in einer - von dem Gebrauch des Absorptionskörpers - ausgetrockneten Klebedispersion vorliegen.

Die Umhüllung hat hierbei verschiedene Funktionen. Obgleich ein technisch nicht allzu aufwendiges Polyprophylen-Nonwoven gewählt sein kann, erfüllt sie viele Funktionen, indem sie Wasserdampf nach außen durchtreten lässt, nachdem sie ihn als flüssiges Exsudat in den Absorptionskörper hat eintreten lassen. Zur Vermeidung der Einsprossung von Gewebe und Gefäßen weist sie Perforationen bzw. Poren auf, die in ihrer Größe derart gewählt sind, dass das Granulationsgewebe der Wunde, insbesondere am Wundrand, nicht in die Umhüllung einsprießen kann. Beispielsweise können die am Umfang der Umhüllung befindlichen Perforationen bzw. Poren kleiner als die des übrigen Umhüllungsbereichs sein.

Die Umhüllung kann aus Naturmaterialien, wie Baumwoll- oder Seidengewebe, oder aus perforierter Kunststoff-Folie bzw. aus Kunststoff-Gewebe gefertigt sein. Die die Matte umgebende Umhüllung kann teilweise oder gänzlich aus einem so genannten Wunddistanzgitter hergestellt sein, wobei die Ausrichtung dessen glatten oder rauen Seite von dem jeweiligen Zweck abhängig ist. Die glatte Seite schützt die Wunde vor Irritation und unerwünschten Einflüssen eines Sekundärverbandes. Eine raue Oberfläche scheint hingegen aktiv bei Bewegung an der Wunde und bewirkt einen erwünschten chemotaktischen Reiz auf die Gewebeneubildung.

Die Umhüllung mit darin untergebrachter Matte sowie die Schaumstoff-Schicht können innerhalb einer äußeren flüssigkeitsdurchlässigen Umhüllung angeordnet sein.

Schließlich kann das Umhüllungsmaterial haftend ausgelegt werden, so dass an der Umhüllung zähflüssige, klebrige, vernetzte oder korpuskuläre Substanzen anhaften und mit dem Wechsel des Absorptionskörpers aus der Wunde heraus befördert werden.

Der Absorptionskörper umfassend die erfindungsgemäße Mischung kann universell für unterschiedliche Wundtherapien (ulcus cruris, Schnittwunden, Schürfwunden, Entzündungswunden, Verbrennungen etc.), Wundheilungsphasen sowie für die Verwendung in Auffangvorrichtungen, wie Drainage-, Stoma-, Fistel- oder anderen Auffangbeuteln angewandt werden.

Nachfolgend sind einige, ausgewählte Verwendungsmöglichkeiten aufgelistet:
- als Verband zur Behandlung einer ödematösen oder entzündlich veränderten Wundregion;
- als Verband zur Behandlung einer mikrobiell belasteten Wundoberfläche, indem über die Saugkraft Keime oder Zelltrümmer mit eingeschlossen, dehydriert oder in anaerobe Gebiete des gequollenen Absorptionskörpers geführt werden;
- als Verband zur Entfernung inflammatorischer Zytokine, Metrixmetalloproteasen, TIMP's, degradiertem Fibronektin (zieht das Gewebe zusammen) oder anderen chronifizierenden Stoffen;
- als Verbandstoff zur Kombination mit schwammartigen, beispielsweise offenporigen Wundbehandlungsmitteln;
- als Verband zur Regulation der Luftfeuchtigkeit, da der Absorptionskörper die wässrigen Bestandteile über deren Dampfdruck wieder in die Luft entlässt;
- als Verband über einem primär applizierten Wunddistanzgitter oder einer Gaze als Sekundärverband ohne unmittelbaren flächenhaften Kontakt zur Wunde;
- als Verband unterhalb einer wasserundurchlässigen Folie, die den Absorptionskörper am Patienten fixiert
- als Verband unterhalb einer wasserdampfdurchlässigen Folie zur Erreichung eines atmungsaktiven Verbandes;
- als Verband bei chronisch venöser Insuffizienz (CVI) mit sekundär nässender Wunde zur Kompression über den Quellvorgang der Leckage des betreffenden Gefäßes;
- als Eingabe in eine Wundbehandlung, die wenigstens temporär subatmosphärische Luftdruckverhältnisse aufweist.

Ausführungsbeispiele der Erfindung sind anhand der Zeichnung erläutert. Die Figuren zeigen:
- Figuren 1a und 1b: einen Absorptionskörper umfassend die erfindungsgemäße Mischung, eingesetzt in der Therapie von ulcus cruris, in einer schematischen Darstellung;
- Fig. 2: Druck- und Sogdiagramme, bezogen auf die Wundsituation gemäß Fig.1;
- Fig. 3: einen zweiten Absorptionskörper umfassend die erfindungsgemäße Mischung, mit einer zusätzlichen inneren Lage, in einer perspektivischen Ansicht;
- Fig. 4: einen Schnitt A-A gemäß Fig. 3;
- Fig. 5: einen anderen Absorptionskörper mit zwei inneren Lagen, in einem schematischen Schnitt;
- Fig. 6: einen Absorptionskörper mit einer zusätzlichen inneren Umhüllung, in einem schematischen Schnitt; und
- Fig. 7: einen weiteren Absorptionskörper, mit einer hydrophilen, inneren Lage aus Carboxymethylcellulose, ebenfalls in einem schematischen Schnitt.

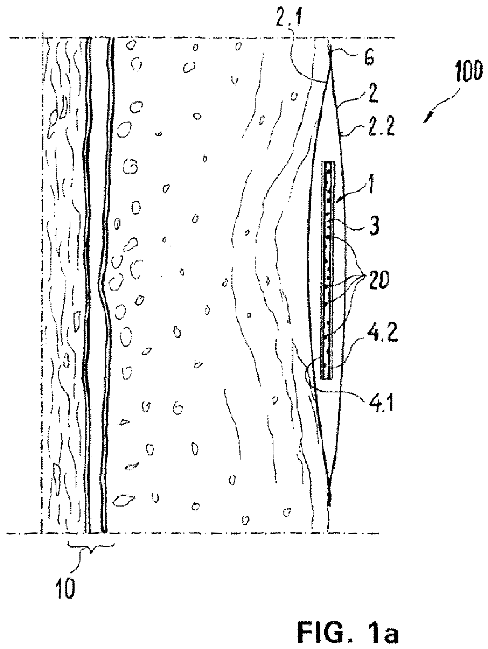

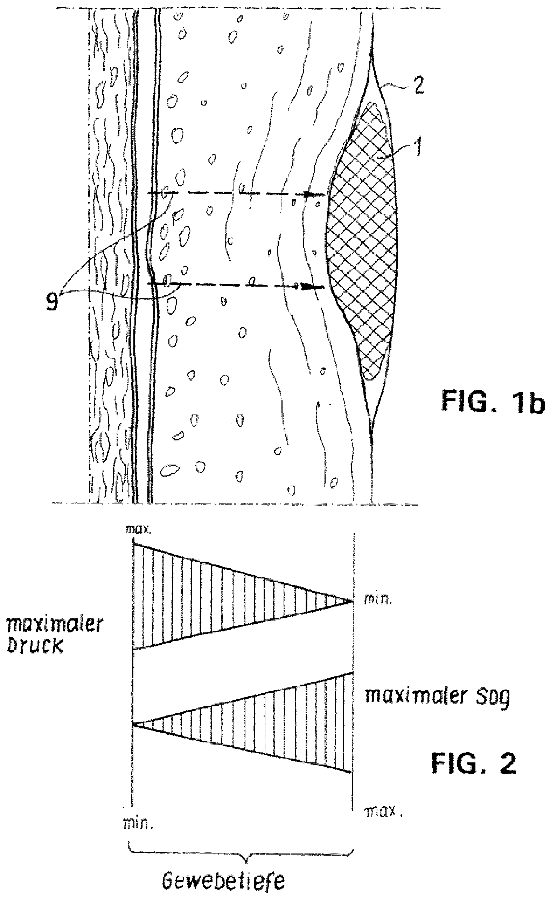

Die Figuren 1a und 1b zeigen schematisch einen Absorptionskörper 100 im Schnitt, bestehend aus einer flüssigkeitsabsorbierenden, inneren Lage 1 und aus einer flüssigkeitsdurchlässigen, perforierten Umhüllung 2. Die innere Lage 1 setzt sich aus einem celluloseartigen Trägermaterial 3 und zwei flächenhaften, ebenfalls celluloseartigen Deckschichten 4.1, 4.2 zusammen, wobei nur der Kern, d. h. das Trägermaterial 3 mit osmotisch wirksamen, superabsorbierenden Polymeren in Form von Superabsorber-Teilchen 20 durchsetzt ist. Die Deckschichten 4.1, 4.2 sind mit dem Trägermaterial 3 kleblos verbunden, d.h., die flächige Verbindung miteinander erfolgte über Presskraft.

Die aus Polypropylenfasern gebildete Umhüllung 2 weist zwei deckungsgleiche, flüssigkeitsdurchlässige, rechteckige Blätter 2.1, 2.2 auf, die miteinander an ihren Rändern über eine Ultraschallnaht 6 so verbunden sind, dass die innerhalb der Umhüllung befindliche Lage 1 im nicht nassen Zustand eine durch die Naht begrenzte Fläche einnimmt, die etwa 75% der Fläche des Blattes 2.1, 2.2 entspricht (vgl. Fig. la). Weiterhin weist die Umhüllung 2 an ihrer Peripherie weiche Kanten 7 auf, die durch Überschreitung der Nahtfläche mit Umhüllungsmaterial entstanden sind.

Zwar ist das celluloseartige Material der inneren Lage 1 flüssigkeitsabsorbierend, jedoch erst eine empirisch festgelegte Vermengung dieses Materials mit Superabsorber-Teilchen 20 ergibt zufriedenstellende Resultate. Dementsprechend wurde das vliesartige Trägermaterial 3 so gefüllt, gelegt und präpariert, dass es zusammen mit der darin enthaltenen pulver- und granulatförmigen Superabsorber- Teilchen 20 eine Flächenmasse von etwa 430 g/m² aufwies, wobei der Anteil an Superabsorber-Teilchen 20 im Trägermaterial 3 54Gew.-% betrug.

Wie in Fig. 1b dargestellt, ist der zuerst im trockenen Zustand auf eine Wunde (ulcus cruris) in das Wundbett aufgelegte Absorptionskörper 100 nach direkter Benetzung mit der Wundflüssigkeit aufgequollen. Es findet über die Funktion des Aufsaugens von Wundflüssigkeit eine Interaktion mit dem Wundbett statt, indem der Absorptionskörper 100 das Wundexsudat speichert, verdampftes Wasser freilässt, Ionen tauscht und darüber ein Gewicht erreicht, welches das Ausmaß der Granulation im Wundbett kontrolliert.

Der Absorptionskörper trennt das in ihn eintretende Wundexsudat auf und lässt Teile dessen unter Maximierung seiner Oberfläche (Tröpfchen der Luftfeuchte) frei. Die innere Lage 1 ändert ihre Dicke entsprechend der lokalen, durch den osmotischen Druck bedingten Sogverhältnisse.

Die Fig. 2 zeigt Druck- und Sogdiagramme, die etwa der Wundsituation gemäß Fig. 1b entsprechen. Im Venenbereich 10 ist der Druck maximal und verringert sich in Richtung Wunde. Der Sog ist wiederum im Venenbereich minimal und vergrößert sich bis zu seinem Maximum im Wundbereich. Die Sogpfeile 9 zeigen die kanalisierte Fließrichtung des Wundexsudat.

Die aufgesogene Flüssigkeit fließt nicht zurück. Der verbrauchte, aufgequollene Absorptionskörper 100 kann mit anhaftenden Teilchen entsorgt werden.

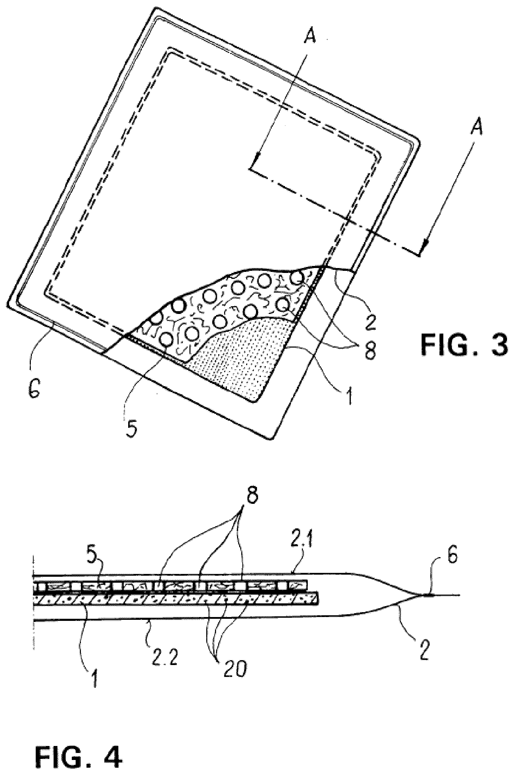

Figuren 3 und 4 zeigen einen zweilagigen Absorptionskörper 200, bei dem die Umhüllung 2 außer der in Fig. 1a gezeigten ersten, mit Superabsorber-Teilchen 20 durchsetzten Lage 1 noch eine zusätzliche, aus Carboxymethylcellulose- Fasern bestehende Lage 5 umgibt. Da der Absorptionskörper 200 mit der Lage 5 auf die jeweilige Wunde einlegbar ist, sind noch an der Lage 5 mehrere durchgehende Öffnungen 8 vorgesehen, die den Transport von Wundflüssigkeiten in Richtung der zweiten, celluloseartigen Lage 1 ermöglichen. Die Öffnungen 8 haben einen Durchmesser von ca. 3 bis 4 mm.

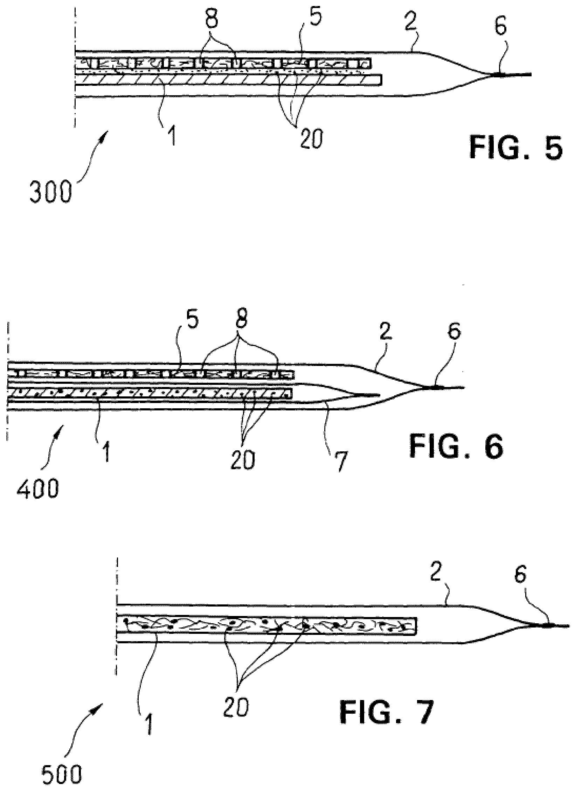

Eine etwas von der in Fig.4 abweichende Ausführungsform (Absorptionskörper 300) ist in Fig. 5 dargestellt. Die Superabsorber-Teilchen 20 liegen verstreut zwischen den beiden inneren Lagen 1 und 5 in einer medizinisch unbedenklichen, ausgetrockneten Klebedispersion, die sich nach der Befeuchtung mit dem Wundexsudat löst. Die Klebedispersion kann optional mit vorgenannten, die Wundheilung fördernden Substanzen angereichert sein.

Weiterhin ist in Fig. 6 ein Absorptionskörper 400 gezeigt, welcher außer der Umhüllung 2 noch eine zweite, innere Umhüllung 7 aufweist. Die beiden Umhüllungen 2; 7 sind flüssigkeitsdurchlässig. Zwischen der inneren Umhüllung 7 und einer Innenfläche 9 der äußeren Umhüllung 2 liegt die durchlochte, aus Carboxymethylcellulose-Fasern bestehende Lage 5. Die innere Umhüllung 7 umgibt die erste, mit Superabsorber-Teilchen 20 durchsetzte Lage 1. Der Absorptionskörper 400 ist ebenfalls mit seiner durchlochten Lage 5 direkt auf die Wunde auflegbar.

Schließlich ist der Fig. 7 ein Absorptionskörper 500 zu entnehmen, bestehend aus der flüssigkeitsdurchlässigen Umhüllung 2 und einer hydrophilen, inneren Lage 1 aus Carboxymethylcellulose 1, die zusätzlich mit Superabsorber-Teilchen 20 durchsetzt ist. Die Umhüllung 2 besteht aus einem perforierten, wundschonenden Folienmaterial, das beispielsweise unter dem Markennamen Tredegar auf dem Markt zugänglich ist und das zur Herstellung von sogenanntem Wunddistanzgitter eingesetzt werden kann. Optional kann die innere Lage 1 durchlocht und gegebenenfalls mit wenigstens einer Deckschicht, wie es bei der Fig. 1a beschrieben worden ist, versehen sein.

## Patentansprüche

1. Mischung aus einer Menge stark osmotisch wirksamer Substanzen, die auf der Basis von Acrylsäure hergestellt sind,
mit einer Menge osmotisch vergleichsweise schwachen oder osmotisch inaktiven Substanzen zur Anwendung in einem Verfahren für die Behandlung chronischer Wunden, der Ödemtherapie oder der Behandlung des Ulcus cruris, zum Aufsaugen von schädlichen Proteasen,
wobei die Mischung in einer inneren Lage (1) vorliegt, die im Wesentlichen aus der Mischung besteht,
wobei die innere Lage (1) von einer äußeren Umhüllung (2), die durchlässig für flüssige Stoffe ist, umschlossen ist,
wobei die innere Lage (1) und die äußere Umhüllung (2) einen Absorptionskörper zur Anbringung an menschliche oder tierische Hautoberflächen im Bereich von Wunden bilden,
wobei die innere Lage (1) derart mit osmotisch wirksamen Substanzen aufgefüllt ist, dass auf eine Wunde mit den darin enthaltenden Wundflüssigkeiten ein osmotischer Druck ausübbar ist, über den die Wundflüssigkeit dem zu behandelnden Organismus entziehbar ist und somit sowohl in der oberflächlichen Wundregion als auch in der Gewebetiefe eine interstitielle Normohydratation von Gewebe unterstützbar ist, indem körpereigene Flüssigkeiten in ihrer Flussrichtung zur Hautoberfläche des Patienten in den Absorptionskörper gelenkt sind, und
und wobei die innere Lage (1) so beschaffen ist, dass die Flächenmasse wenigstens 420 g/m² ist und wobei die Flächenmasse des darin gleichmäßig verteilten Anteils der osmotisch wirksamen Substanzen wenigstens 200 g/m² beträgt.

2. Mischung nach Anspruch 1, wobei die innere Lage (1) zu mehr als 40 Gew.-% aus osmotisch wirksamen Substanzen besteht, bezogen auf das Gesamtgewicht der inneren Lage (1).

3. Mischung nach einem der Ansprüche 1 oder 2, **dadurch gekennzeichnet, dass** die Anwendung die Verwendung bei iatrogenen Durchtrennungsstellen der Haut, Verbrennungswunden, nässenden entzündlichen Prozessen der Haut oder exulzerierenden Prozessen neoplastischer Genese, nässenden Infektionen, Fisteln umfasst.

4. Mischung nach einem der vorangehenden Ansprüche, **dadurch gekennzeichnet, dass** der Absorptionskörper als Verband als Teil einer Kompressionstherapie, als Teil einer Therapie bei Kompartmentsyndrom oder bei der Vakuumtherapie verwendet wird.

5. Mischung nach einem der vorangehenden Ansprüche, **dadurch gekennzeichnet, dass** die osmotisch vergleichsweise schwache oder osmotisch inaktive Substanz Zellulose ist.

6. Mischung nach einem der vorangehenden Ansprüche, **dadurch gekennzeichnet, dass** die osmotisch wirksamen Substanzen in Pulver- oder Granulatform vorliegen.

7. Mischung nach einem der vorangehenden Ansprüche, **dadurch gekennzeichnet, dass** die Umhüllung (2) aus zwei Blättern (2.1, 2.2) besteht, die an ihrer Peripherie miteinander verbunden sind.

8. Mischung nach einem der vorangehenden Ansprüche, **dadurch gekennzeichnet, dass** die Umhüllung (2) Poren aufweist, die jeweils kleiner als die Ausmaße der zu absorbierenden Substanzen in Granulat- oder Pulverform sind.

9. Mischung nach einem der vorangehenden Ansprüche, **dadurch gekennzeichnet, dass** die Umhüllung (2) haftend für zähflüssige, klebrige, vernetzte oder korpuskuläre Substanzen ist, die somit bei Wechsel des Absorptionskörpers aus der Wunde herausförderbar sind.

10. Mischung nach einem der vorangehenden Ansprüche, **dadurch gekennzeichnet, dass** die innere Lage (1) aus einer Zwischenschicht, bestehend aus einer Mischung von superabsorbierenden Polymeren mit Cellulose als Trägermaterial (3), und aus zwei flächenhaften Deckschichten (4.1, 4.2) gebildet ist.

11. Mischung nach einem der vorangehenden Ansprüche, **dadurch gekennzeichnet, dass** die innere Lage (1) aus hydrophilen Fasern, wie Carboxymethylcellulose-Fasern, und superabsorbierenden Polymeren mit oder ohne Cellulose besteht.

12. Mischung nach einem der vorangehenden Ansprüche, **dadurch gekennzeichnet, dass** die innere Lage (1) derart zusammengesetzt ist, dass im auf die Wunde aufgelegten Zustand des Absorptionskörpers die Wiederfreigabe von Flüssigkeit erst im Sättigungsbereich eintritt.

13. Mischung nach einem der vorangehenden Ansprüche, **dadurch gekennzeichnet, dass** mit der inneren Lage (1) über Wundflüssigkeiten transportierte gewebe- und wachstumsschädigende Proteasen und Faktoren lokal fixierbar sind.

14. Mischung nach einem der vorangehenden Ansprüche, **dadurch gekennzeichnet, dass** die Proteasen Matrix Metallo Proteasen oder TIMPs (Tissue Inhibiting Metallo Proteasen) sind.

## Claims

1. Mixture of a quantity of strongly osmotically active substances prepared on the basis of acrylic acid
and a quantity of osmotically comparatively weak or osmotically inactive substances, for use in a method for treating chronic wounds, edema therapy or treating leg ulcers, in order to take up harmful proteinases, wherein the mixture is present in an inner layer (1) which substantially consists of the mixture,
wherein the inner layer (1) is enclosed by an outer covering (2) which is permeable to liquids,
wherein the inner layer (1) and the outer covering (2) form an absorbent body for applying to human or animal skin surfaces in the region of wounds, wherein the inner layer (1) is filled with osmotically active substances in such a way that an osmotic pressure can be exerted on a wound having the wound exudates contained therein, by means of which osmotic pressure the wound exudate can be drawn away from the organism to be treated and interstitial normohydration of tissue can thus be supported both in the superficial wound region and in the tissue depth, by directing body fluids into the absorbent body in the flow direction thereof towards the skin surface of the patient, and
and wherein the inner layer (1) is structured such that the area density is at least 420 g/m² and wherein the area density of the proportion of the osmotically active substances uniformly distributed therein is at least 200 g/m².

2. Mixture according to claim 1, wherein the inner layer (1) consists of more than 40 wt.% of osmotically active substances, based on the total weight of the inner layer (1).

3. Mixture according to any of claims 1 or 2, **characterized in that** the application comprises the use in iatrogenic skin transections, burn wounds, weeping inflammatory processes of the skin or ulcerating processes of neoplastic origin, weeping infections, fistulas.

4. Mixture according to any of the preceding claims, **characterized in that** the absorbent body is used as a dressing as part of compression therapy, as part of therapy for compartment syndrome or in vacuum therapy.

5. Mixture according to any of the preceding claims, **characterized in that** the osmotically comparatively weak or osmotically inactive substance is cellulose.

6. Mixture according to any of the preceding claims, **characterized in that** the osmotically active substances are in powdered or granular form.

7. Mixture according to any of the preceding claims, **characterized in that** the covering (2) consists of two sheets (2.1, 2.2) which are connected together at the periphery thereof.

8. Mixture according to any of the preceding claims, **characterized in that** the covering (2) has pores which are each smaller than the dimensions of the granular or powdered substances to be absorbed.

9. Mixture according to any of the preceding claims, **characterized in that** the covering (2) adheres to viscous, sticky, cross-linked or corpuscular substances which can therefore be extracted from the wound when the absorbent body is changed.

10. Mixture according to any of the preceding claims, **characterized in that** the inner layer (1) is formed from an intermediate layer consisting of a mixture of superabsorbent polymers having cellulose as a carrier material (3), and from two planar cover layers (4.1, 4.2).

11. Mixture according to any of the preceding claims, **characterized in that** the inner layer (1) consists of hydrophilic fibers, such as carboxymethylcellulose fibers, and superabsorbent polymers with or without cellulose.

12. Mixture according to any of the preceding claims, **characterized in that** the inner layer (1) is composed in such a way that, when the absorbent body is placed on the wound, the re-release of fluid occurs only in the saturation region.

13. Mixture according to any of the preceding claims, **characterized in that** tissue- and growth-damaging proteinases and factors transported via wound exudates can be locally fixed by means of the inner layer (1).

14. Mixture according to any of the preceding claims, **characterized in that** the proteinases are matrix metalloproteinases or TIMPs (tissue inhibiting metalloproteinases).

## Revendications

1. Mélange d'une quantité de substances à fort pouvoir osmotique fabriquées à base d'acide acrylique,
comportant une quantité de substances osmotiquement relativement faibles ou osmotiquement inactives, pour utilisation dans un procédé de traitement pour plaies chroniques, de thérapie pour œdème ou de traitement de l'ulcère de la jambe, permettant d'absorber des protéases nocives,
dans lequel le mélange est présent dans une couche intérieure (1) qui est sensiblement constituée du mélange,
dans lequel la couche intérieure (1) est entourée d'une enveloppe extérieure (2) qui est perméable aux substances liquides,
dans lequel la couche intérieure (1) et l'enveloppe extérieure (2) forment un corps absorbant destiné à être appliqué sur des surfaces de la peau humaine ou animale dans la région de plaies,
dans lequel la couche intérieure (1) est remplie de substances à pouvoir osmotique de telle sorte qu'une pression osmotique peut être exercée sur une plaie avec les liquides de la plaie qu'elle contient, par l'intermédiaire de laquelle le liquide de la plaie peut être extrait de l'organisme à traiter et une normo-hydratation interstitielle des tissus peut ainsi être soutenue aussi bien dans la région superficielle de la plaie que dans la profondeur des tissus, dans lequel des liquides propres à l'organisme sont dirigés dans le corps absorbant dans leur direction d'écoulement vers la surface de la peau du patient, et
dans lequel la couche intérieure (1) est telle que la masse surfacique est d'au moins 420 g/m² et dans lequel la masse surfacique de la partie des substances à pouvoir osmotique qui y sont réparties uniformément est d'au moins 200 g/m².

2. Mélange selon la revendication 1, dans lequel la couche intérieure (1) est constituée à plus de 40 % en poids de substances à pouvoir osmotique par rapport au poids total de la couche intérieure (1).

3. Mélange selon l'une des revendications 1 ou 2, **caractérisé en ce que** l'application comprend l'utilisation sur des emplacements de sectionnement iatrogènes de la peau, des plaies de brûlure, des processus inflammatoires suintants de la peau ou des processus d'exulcération d'origine néoplasique, des infections suintantes, des fistules.

4. Mélange selon l'une des revendications précédentes,
**caractérisé en ce que** le corps absorbant est utilisé comme bandage dans le cadre d'une thérapie par compression, dans le cadre d'une thérapie en cas de syndrome des loges ou dans le cadre d'une thérapie par le vide.

5. Mélange selon l'une des revendications précédentes,
**caractérisé en ce que** la substance osmotiquement relativement faible ou osmotiquement inactive est la cellulose.

6. Mélange selon l'une des revendications précédentes,
**caractérisé en ce que** les substances à pouvoir osmotique se présentent sous forme de poudre ou de granulés.

7. Mélange selon l'une des revendications précédentes,
**caractérisé en ce que** l'enveloppe (2) est constituée de deux feuilles (2.1, 2.2) reliées entre elles au niveau de leur périphérie.

8. Mélange selon l'une des revendications précédentes,
**caractérisé en ce que** l'enveloppe (2) présente des pores qui sont respectivement plus petits que les dimensions des substances à absorber sous forme de granulés ou de poudre.

9. Mélange selon l'une des revendications précédentes,
**caractérisé en ce que** l'enveloppe (2) est adhésive pour des substances visqueuses, collantes, réticulées ou corpusculaires, qui peuvent ainsi être transportées hors de la plaie lors du changement du corps absorbant.

10. Mélange selon l'une des revendications précédentes,
**caractérisé en ce que** la couche intérieure (1) est formée d'une couche intermédiaire, constituée d'un mélange de polymères superabsorbants avec cellulose en tant que matériau formant support (3), et de deux couches de recouvrement (4.1, 4.2) extensives.

11. Mélange selon l'une des revendications précédentes,
**caractérisé en ce que** la couche intérieure (1) est constituée de fibres hydrophiles, telles que des fibres de carboxyméthylcellulose, et de polymères superabsorbants avec ou sans cellulose.

12. Mélange selon l'une des revendications précédentes,
**caractérisé en ce que** la couche intérieure (1) est composée de telle sorte que, dans l'état du corps absorbant appliqué sur la plaie, la libération du liquide n'intervient que dans la zone de saturation.

13. Mélange selon l'une des revendications précédentes,
**caractérisé en ce que** des protéases et des facteurs nuisibles aux tissus et à la croissance, transportés par l'intermédiaire des liquides de la plaie, peuvent être fixés localement avec la couche intérieure (1).

14. Mélange selon l'une des revendications précédentes,
**caractérisé en ce que** les protéases sont des métalloprotéases matricielles ou des TIMP (Tissue Inhibiting Metallo Proteasen).
